Europäisches Patentamt

European Patent Office

Office européen des brevets

(19)

(11) Publication number: **0 139 745 B1**

# EUROPEAN PATENT SPECIFICATION
## published in accordance with Art. 158(3) EPC

(12)

(45) Date of publication of patent specification: **21.08.91**

(51) Int. Cl.⁵: **A61F 5/00, A61F 2/26**

(21) Application number: **84901872.6**

(22) Date of filing: **17.04.84**

(86) International application number:
**PCT/US84/00585**

(87) International publication number:
**WO 84/04035 (25.10.84 84/25)**

(54) PENILE PROSTHESIS.

(30) Priority: **18.04.83 US 485647**
        **18.04.83 US 485648**

(43) Date of publication of application:
**08.05.85 Bulletin 85/19**

(45) Publication of the grant of the patent:
**21.08.91 Bulletin 91/34**

(84) Designated Contracting States:
**DE FR GB SE**

(56) References cited:
**DE-A- 3 027 681**
**GB-A- 2 151 484**
**US-A- 4 545 081**

(73) Proprietor: **DACOMED CORPORATION**
**1701 East 79th Street Suite 17**
**Minneapolis, MN 55420(US)**

(72) Inventor: **TIMM, Gerald, W.**
**29822 Highview Circle**
**San Juan Capistrano, CA 91675(US)**
Inventor: **SANFORD, Donald, W.**
**1698 Eustis Street**
**Lauderdale, MN 55113(US)**
Inventor: **HELMS, Richard, A.**
**863 Vernon Avenue**
**Elk River, MN 55330(US)**
Inventor: **CLAUDE, Timothy, J.**
**445 10th Lane**
**Coon Rapids, MN 55433(US)**

(74) Representative: **Ström, Tore et al**
**Ström & Gulliksson AB Studentgatan 1 P.O.**
**Box 4188**
**S-203 13 Malmö(SE)**

## Description

### Background of the Invention

This invention relates to the treatment of erectile impotence. More particularly, the present invention relates to a penile prosthesis which may be implanted in a flaccid penis for enabling the achievement of an erectile state.

The causes of male impotence are many and varied. Various approaches to treating impotence have been developed over the past two decades. Penile prostheses implanted within the penis to simulate an erectile state are shown for example in US-A-3,987,789. That patent shows a prosthesis including an elongated malleable rod portion housed within a generally tubular physiologically inert plastic body. The malleable rod portion enables the prosthesis to be conformed to a variety of shapes by bending or twisting. During intercourse the prosthesis will maintain the penis in an erectile state and afterwards the penis may be positioned and maintained by the user in a convenient, comfortable position. The prosthesis depends upon its malleability to permit moving the penis to a convenient comfortable position. The flexibility and similar characteristics of the prosthesis are not controllable by the patient.

US-A-3,954,102 shows a penile erection system having two conditions. In one condition, the implanted prosthesis is controlled by varying the amount of fluid in cylinders within the prosthesis by squeezing an elastomeric bulb through the patient's skin to transfer fluid from a reservoir into cylinders in the prosthesis. Because the reservoir and bulb are positioned outside of the prosthesis within the patient's body, the implantation procedure is quite complex and the extensive tubing required to interconnect the various portions of the system increase the possibility of failure of the system.

Other implantable prostheses have been developed which incorporate a reservoir pump and valving into the prothesis itself as shown for example, in US-A-4,369,771 and US-A-4,353,360. Those systems still require pump and valving apparatus to be installed within the prosthesis and require the transfer of fluid from a reservoir into an inflatable portion for operation.

The present invention solves these and many other problems associated with currently available devices.

### Summary of the Invention

The present invention relates to a penile prosthesis for implantation in a penis. The prosthesis comprising an articulated column of segments engaging each other to form slidable joints, an outer elongated sheath of a physiologically inert and pliable material extending longitudinally of the prosthesis and connected to the prosthesis proximate to both ends of the prosthesis for holding the segments of the articulated column to interengagement at adjacent ends thereof, the prosthesis being adjustable between flaccid and rigid conditions.

A prosthesis of this kind is disclosed in US-A-4,151,840.

According to the invention the prosthesis has obtained the characterizing features of claim 1.

The prosthesis of the present invention is particularly advantageous in that it can be implanted surgically without regard to angular orientation thereby avoiding the possibility of failure if during implant or in use, the prosthesis partially rotates about its longitudinal axis. Accordingly, the prosthesis is designed to be generally symmetrical about its longitudinal axis.

Furthermore, the present invention provides for volitional control of erection, generating sufficient stiffness of the penis for intercourse, and permits user deactivation, whereby the penis recovers as a flaccid state.

Yet another advantageous feature of the present invention is that it readily enables patient activation and deactivation.

Furthermore, the present invention is designed to be implanted by standard surgical procedures and is biologically compatible with the human body environment.

In addition, the present invention provides for activation/deactivation over many cycles of use.

Furthermore, the present invention enables the penis to obtain a flaccid-like state when the prosthesis is deactivated and provides sufficient rigidity for intercourse when the prosthesis is activated.

The prosthesis of the present invention is bistable. In other words it operates in either a flexible or a stiffened mode at the user's control. Activation and deactivation is achieved by manual bending of the penis, whereupon the prosthesis is alternately rigid or flaccid. Bending of the prosthesis places the tension means in tension which causes the switch means to be axially displaced in a proximal direction, the locking means alternately securing the prosthesis in a deactivated position.

When the prosthesis is activated, the increased tension in the tension means and the compression of the articulated column induces a stress state in the prosthesis that displays bending stiffness. This is attributable to: (1) Interfacial friction between the segments of the column; (2) tension in the tension means; and (3) friction between the sheath and the internal articulated column along the cylindrical circumference of the prosthesis.

The penile prosthesis, when implanted longitudinally in the corpora cavernosa of the penis is switched from one condition to another by repetitive compressive palpations of the penis at activation or deactivation sites readily locatable by finger palpation of the implanted prosthesis. The prosthesis characteristics are controlled by the switch means. Compressive palpation of the activation site causes the switch to longitudinally extend itself against the column of segments until the increased frictional resistance between the joints results in a relatively rigid penis.

For a better understanding of the invention, its advantages, and objects attained by its use, reference should be had to the drawings which form a further part hereof, and to the accompanying descriptive matter, in which there is illustrated and described a preferred embodiment of the invention.

Brief Description of the Drawings

In the drawings, in which like reference numerals and letters indicate corresponding parts throughout the several views:

FIGURE 1 is a longitudinal axial sectional view of a preferred embodiment of the present invention in a deactivated or flaccid state;

FIGURE 2 is a longitudinal sectional view of the embodiment shown in FIGURE 1 in an activated or rigid state;

FIGURE 3 is an enlarged axial sectional view with portions broken away of the switch apparatus of the embodiment shown in FIGURE 2 in an activated state;

FIGURE 4 is a further enlarged sectional view of a portion of the switch apparatus shown in FIGURE 3 with parts thereof removed for clarity of illustration;

FIGURE 5 is a transverse sectional view as generally seen along line 5-5 of FIGURE 3;

FIGURE 6 is a longitudinal view of an alternate embodiment of the present invention wherein an outer sheath is operatively interconnected to the switch apparatus;

FIGURE 7 is a fragmentary longitudinal axial sectional view of yet another embodiment of the present invention in a flaccid state;

FIGURE 8 is a fragmentary longitudinal axial sectional view of the embodiment shown in FIGURE 7 in an activated state;

FIGURE 9 is an enlarged fragmentary sectional view of a switch apparatus of the embodiment shown in FIGURE 7 in a deactivated state;

FIGURE 10 is a fragmentary longitudinal axial sectional view of still another embodiment of the present invention; and

FIGURE 11 is yet another embodiment of the present invention.

Detailed Description of a Preferred Embodiment of the Present Invention

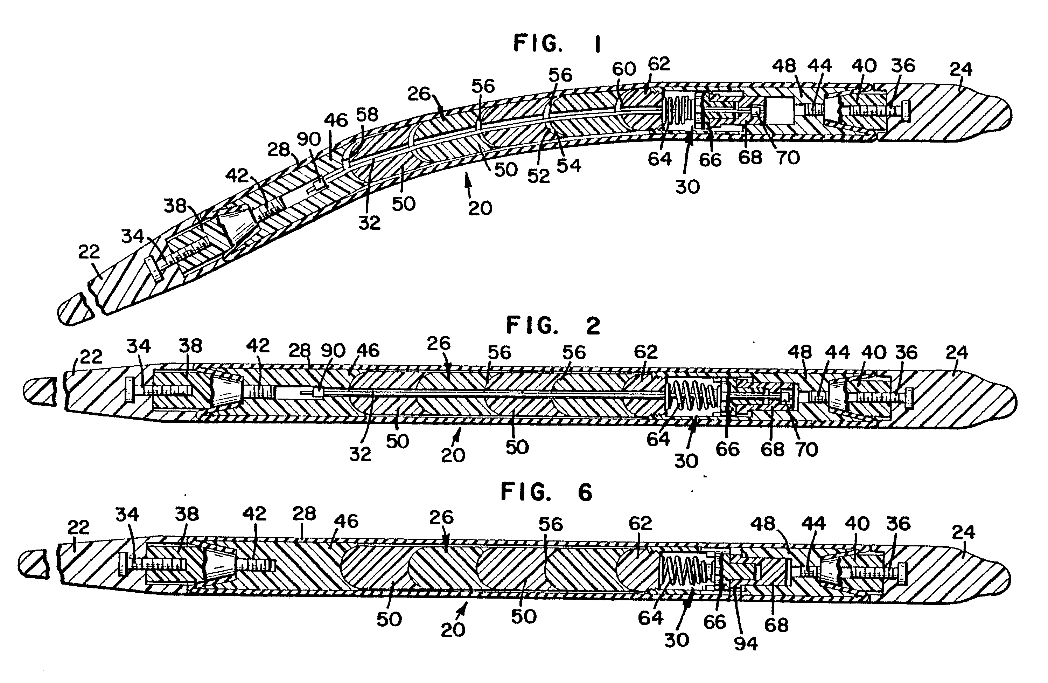

Referring now to the drawings, a preferred embodiment of a mechanical penile prosthesis in accordance with the principles of the present invention is illustrated in Figures 1 and 2, the penile prosthesis generally being referred to by the reference numeral 20. The prosthesis 20 is generally shown as an elongated member including a proximal end portion 22 and a distal end portion 24. An elongated articulated column 26 is positioned between the proximal and distal end portions 22 and 24 enabling pivotal or bending motion of the prosthesis 20 in all directions (360 degrees) about the longitudinal axis of the prosthesis 20. Interposed between the distal end of the articulated column 26 and the distal end portion 24 is a switch apparatus 30. The articulated column 26 and the switch apparatus 30 are axially journaled about an axially extending elongated tension member 32 which extends from proximate the proximal end of the articulated column 28 to proximate the distal end of the switch apparatus 30. The articulated column 26 and the switch apparatus 30 are enclosed by a sheath 28 of physiologically inert and pliable material which shields the prosthesis 20 from body fluids so as to prevent their interference with the functioning of the prosthesis 20.

The switch apparatus 30 in cooperation with the tension member 32 and the articulated column 26 provides the embodiment of the present invention with bistable characteristics, i.e. the prosthesis 20 may be manually activated to a rigid/activated state as generally illustrated in Figure 2 or deactivated to a flexible/flaccid state as generally indicated in Figure 1. The prosthesis 20 remains in either of these two states until manually activated or deactivated.

The prosthesis 20 of the present invention is designed for implantation in the corpora of the penis by standard surgical procedures for treatment of erectile impotence. The prosthesis 20 is configured to generally match penile corpora size so as to extend sufficiently proximally and distally when anchored within the penis and body cavity so as to induce an erected penile state generating sufficient stiffness of the penis for intercourse when activated and to provide the penis with flaccid characteristics when deactivated.

Accordingly, the present invention provides for volitional control of penile erection and return thereof to a flaccid state. Furthermore, the configuration of the present invention enables surgical implantation without regard for angular orientation and prevents malfunction if angular rotation is realized during use. In addition, the present invention is biologically compatible with the human body environment and enables activation/deactivation over many repetitive cycles of use.

More particularly, the proximal end portion 22 is tapered to assist in anchoring the prosthesis 20 in the body and the distal end portion 24 is bullet- or cone-shaped to adapt to the distal end of the penis. The proximal end portion 22 and the distal end portion 24 are provided in various lengths, preferably ranging from one to four centimeters. The appropriate length for each is selected at the time of implant as determined by the incision site and by the patient's total intracorporeal length. Preferably, the proximal end portion 22 and the distal end portion 24 are made from a semi-rigid material such as silicon rubber.

The proximal end portion 22 and the distal end portion 24 are attached to the segmented, flexible body of the prosthesis 20. This might be accomplished in any of several ways such as by a positive locking, snap-on mechanism (not shown) or by threaded attachment of the end portions 22, 24 to the flexible body of the prosthesis 20. This is accomplished in the preferred embodiment as generally illustrated in Figures 1 and 2 by elongated threaded members 34, 36 securely embedded in the proximal end portion 22 and the distal end portion 24, respectively, which are threaded into clamp members 38, 40, respectively. The clamp members 38, 40 in turn are threadedly attached to the flexible body of the prosthesis by elongated threaded members 42, 44, respectively. The clamp members 38, 40 cooperate with members 46, 48, which are configured to receive the clamp members 38, 40 such that the end portions of the sheath 28 are wedged between the clamp members 38, 40, and the inside sloping walls of the members 46, 48. Accordingly, the sheath 28 is retained in position at the proximal and the distal end portions of the prosthesis 20.

The articulated column 26 comprises a plurality of cylinders 50. Each of the cylinders 50 has a concave surface 52 and a convex surface 54, the cylinders 50 being oriented such that the adjacent surfaces of the cylinders cooperate to form ball and socket joints 56. The articulated column 26 is journaled axially to receive the tension member 32. The cylinders 50 as well as the other internal parts of the prosthesis are made from a surgically implantable material, preferably an implantable plastic, such as polyurethane.

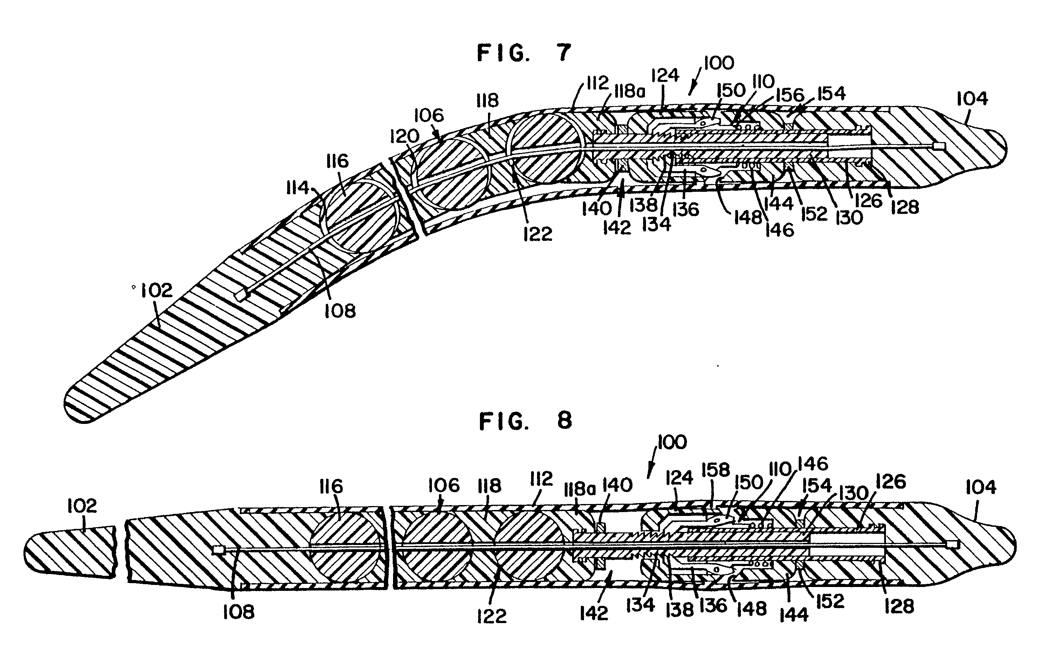

It will be appreciated that in alternate embodiments of the present invention, other elements with slidable cooperating surfaces may be utilized to form an articulated column. For example, as illustrated in Figures 7 and 8, the articulated column might be formed of alternating cylinders and spheres forming interfitting ball and socket joints. Yet another example is that of U. S. Patent No. 4,151,840 (Barrington) wherein the cylinders of the articulated column have modified convex and concave surfaces.

In the embodiment illustrated, the cylindrical member 46 to which the sheath 28 is clamped, has a generally concave surface 58 adjacent the proximal end of the articulated column 26 to form a ball and socket joint with the articulated column 26.

Furthermore, a proximal end portion 62 of the cylindrical member 48 has a convex surface 60 thereby forming a ball and socket joint with the articulated column 26.

Interposed between the distal end of the articulated column 26, and the distal end portion 24 is the switch apparatus 30. As previously discussed, in the preferred embodiment shown, the cylindrical member 48 which cooperates with the clamp member 40 in retaining the sheath 28 at the proximal end of the prosthesis 20, also functions as the housing for the switch apparatus 30. The proximal end portion 62 of the housing 48 is illustrated as being theadedly attached to the housing 48 to facilitate assembly of the switch apparatus 30.

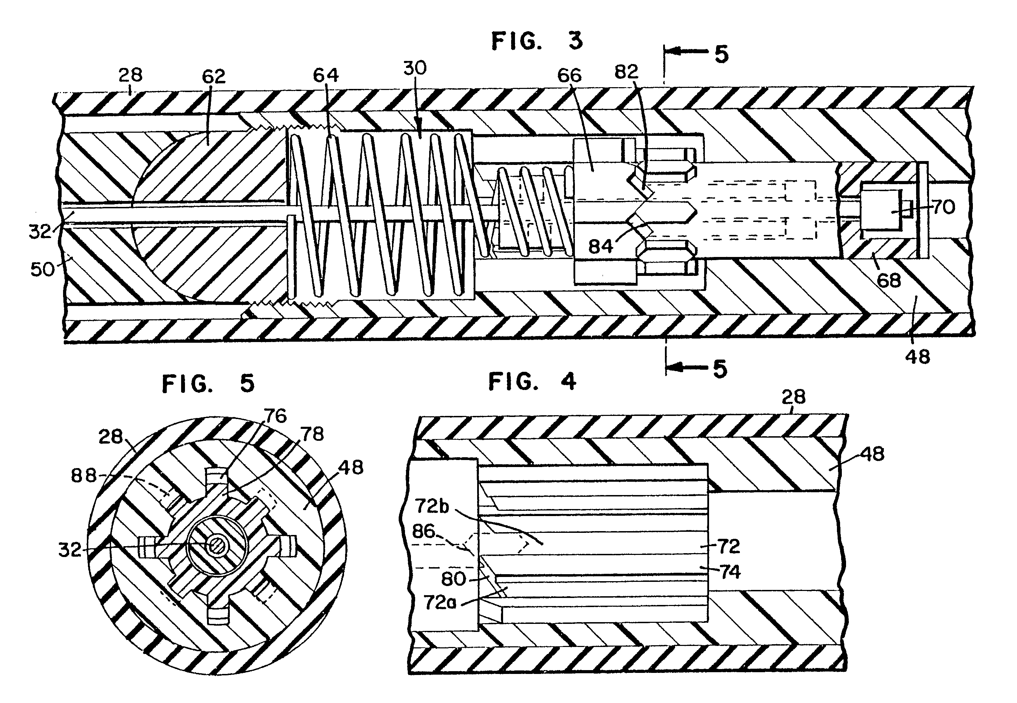

As illustrated in Figure 3, positioned within a cavity of the housing 48 are a coil spring 64, a rotary actuator 66, and a sliding actuator 68 which is securedly attached to the distal end of the tension member 32 at location 70. As further illustrated in Figures 3-5, the inside surface of the housing 48 includes a plurality of axially extending alternating grooves 72 and ridges 74 along a portion thereof, which serve as guides for spaced-apart ridges 76, 78 positioned about the circumference of the actuators 66, 68, respectively so as to maintain the relative positioning of the rotary actuator 66 with respect to the sliding actuator 68. Furthermore, the proximal ends of the grooves 72 and ridges 74 are oblique with respect to the longitudinal axis such that an adjacent groove and ridge cooperate to form a continuous sloping surface 80. The adjoining end portions 82, 84 of the rotary actuator 66 and the sliding actuator 68 have a saw-toothed configuration to form a gear-like meshing therebetween. The spaced apart ridges 76, 78 about the circumference of the rotary actuator 66 and the sliding actuator 68 are positioned such that when the actuators 66, 68 are positioned in the grooves 72 of the housing 48, the end portions 82, 84 of the actuators are offset or staggered as generally illustrated in Figure 3. Accordingly, when the rotary actuator 66, is positioned outside of the housing grooves 72, the rotary actuator 66 will rotate about the longitudinal axis so as to align itself with the saw-toothed configuration of the sliding actuator end portion 84 as illustrated by the broken lines 86 in Figure 4. When the rotary actuator 66 moves toward the distal end of the prosthesis 20, the oblique surfaces 80 of each of the cooperating housing ridges 74 and grooves 72 will cause the rotary actuator 66 to again rotate about the longitudinal axis of the prosthesis 20 such that the rotary actuator 66 is once again offset from the saw-toothed configuration of the sliding actuator end portion 84.

In addition, as illustrated in Figure 5, while there are eight of the housing grooves 72, alternating ones of the grooves 72a do not extend radially as far as the others of the grooves 72b. Furthermore, as illustrated in Figure 5, the ridges 76 of the rotary actuator 66, there being four in number, extend further radially than the grooves 72a, while the ridges 78, there being eight in number, of the sliding actuator 68 do not extend radially as far as the grooves 72a. The rotary actuator 66 and the sliding actuator 68 are configured such that when the rotary actuator 66 extends beyond the grooves 72 it will rotate one-eighth of a revolution such that the rotary actuator ridges 76 will alternately engage the housing grooves 72a as generally illustrated by the broken lines 88 in Figure 5 and alternately slide toward the distal end of the prosthesis in the grooves 72b. Accordingly, the rotary actuator 66 is alternately locked in a deactivated position wherein the prosthesis 20 is generally flaccid and unlocked so as to move distally of the prosthesis into an activated position wherein the prosthesis 20 is relatively rigid.

The coil spring 64 positioned between the proximal end portion 62 of the housing and the rotary actuator 66 biases the actuators 66, 68 toward the distal end of the prosthesis 20. As previously indicated, the tension member 32 is attached to the sliding actuator 68 at the location 70 and is further attached to the member 46 at location 90. Accordingly, the biasing effect of the coil spring 64 is overcome by placing the axial tension member 32 in sufficient tension such that the force exerted by the rotary actuator 66 and the sliding actuator 68 on the coil spring 64 is greater than that of the coil spring 64, whereby the rotary actuator 66 and the sliding actuator 68 are caused to move toward the proximal end of the prosthesis 20, thereby causing the rotary actuator 66 to rotate upon moving beyond the housing grooves 72. The tension member 32, which is preferably prestretched prior to assembly, is placed in tension by bending the prosthesis 20 due to its fixed length. The applicant has found that a tension member made from braided stainless steel provides the preferred characteristics of lightweight, strength, and resistance to the corrosive effects of body fluids.

It will be appreciated that the above described switch apparatus is not unlike that of a ballpoint pen actuator mechanism and that varying embodiments of the switch apparatus 30 in keeping with the principles of the present invention might be utilized.

As previously indicated, the entire prosthesis 20 is covered with the sheath 28 of physiologically inert and pliable material to shield the prosthesis from body fluids and prevent interference with its functioning, the sheath 28 being wedgedly secured by the clamp members 38, 40 in cooperation with the members 46, 48 near the proximal and distal ends of the prosthesis 20. The applicant has found that expanded polytetrofloralethylene (PTFE) such as the product sold by Dupont Corporation under the trademark Teflon is a suitable material.

In use, the implanted prosthesis 20 is normally maintained in an untensioned or flaccid state which does not interfere with bending or moving of the penis. Accordingly, the rotary actuator 66 is locked in its deactivated position such that the tension member 32 is not in tension. This lack of tension allows the surfaces 52, 54 of the cylindrical members 50 forming the ball and socket joints 56 of the acticulated column 26, to freely slide relative to each other under low friction conditions thereby providing the prosthesis 20 and thus the penis with flexibility as though in a flaccid, non-erect state.

To provide an erection, the user manually bends the prosthesis 20 which results in increased tension in the fixed length tension member 32. When the force exerted by the tension member 32 exceeds that of the coil spring 64, the rotary actuator 66 and the sliding actuator 68 are displaced axially toward the proximal end of the prosthesis 20. As previously described, configuration of the rotary actuator 66 and the sliding actuator 68 is such that the rotary actuator 66 is caused to rotate about its axis when axially displaced a predetermined amount, whereby the rotary actuator 66 is unlocked from its deactivated state. Accordingly, when the prosthesis is straightened, the rotary actuator 66 and the sliding actuator 68 are axially displaced toward the distal end of the prosthesis 20 by the coil spring 64 to a location closer to the distal end of the prosthesis 20 then when in the deactivated state. Accordingly, a degree of tension is induced in the tension member 32 and the articulated column 26 is compressed due to the biasing effect of the coil spring 64. As a result of the increased tension and compression of the articulated column 26, the frictional force between the radiused ends of the cylindrical members 50 is increased such that the prosthesis 20 and correspondingly the penis are in a rigid or erect state. Furthermore, friction between the sheath 28 and the articulated column 26 along the longitudinal circumference contributes to the overall rigidity or stiffness of the prosthesis 20.

Subsequent restoration of the penile flaccidity or flexibility is achieved by again bending the prosthesis 20 into a curved shape. Tension in the tension member 32 is increased to a point where it exerts a force which exceeds that of the biasing effect of the coil spring 64, whereby the rotary actuator 66 and the sliding actuator 68 are axially displaced in a proximal direction. The rotary actuator 66 is caused to rotate about its axis upon moving axially beyond the grooves 72 in the housing 48 such that the rotary actuator 66 is again locked in a deactivated position at a location closer to the proximal end of the prosthesis 20 than when in the activated state. Accordingly, tension in the tension member 32 is again reduced enabling the ball and socket joints 56 of the articulated column 26 to exhibit a high degree of freedom of movement such that the prosthesis 20 and correspondingly the penis is again in the flaccid state.

Subsequent bending and unbending may be carried out for numerous repetitions with the prosthesis 20 being alternately rigid and flaccid. The prosthesis 20 thus exhibits bistable characteristics in that it maintains either of the states until being manually changed.

An alternate embodiment of the present invention which does not utilize the axial tension member 32 is illustrated in Figure 6. In this embodiment, the sliding actuator 68 of the switch apparatus 30 is attached to the sheath 28. Accordingly, the sheath 28 is alternately placed in tension and loosened upon bending of the prosthesis 20. In this manner, the prosthesis 20 is alternately placed in a rigid or flaccid state by a procedure not unlike that of the previous embodiment. In the embodiment illustrated, sliding actuator 68 is attached at 94 to the sheath 28 although it will be appreciated that various methods of attachment to the sliding actuator 30 in keeping with the principles of the present invention might be utilized.

In this embodiment, some, such as alternating ones, or all of the segments of the articulated column might be made of a softer material. Accordingly, upon compression of the articulated column the softer segments will bulge outwardly against the sheath 28 so as to provide additional frction and rigidity.

Figures 7-9 illustrate yet another embodiment of the present invention generally designated by the reference numeral 100. The prosthesis 100 includes a proximal end portion 102 and a distal end portion 104. An articulated column 106, somewhat like that of the previous embodiments, is journaled axially about a tension member 108 and is positioned between the proximal end portion 102 and the distal end portion 104. A switch apparatus 110, somewhat unlike that of the previous embodiments, is interposed between the distal end portion 104 and the articulated column 106. The prosthesis 100, as with the previous embodiments, is enclosed by a sheath 112 of physiologically inert and pliable material to shield the prosthesis from body fluids and prevent interference with its functioning. Sheath 112 also may be utilized to assist in providing rigidity to the prosthesis 100 when in an activated or rigid state. As with the previous embodiments, the prosthesis 100 is manually switchable from an activated or rigid state to a deactivated or flaccid state.

More particularly, the tension member 108 extends from the proximal end portion 102 wherein it is securely embedded to the distal end portion 104 wherein it is also securely embedded. The proximal end portion 102 is configured to function as an anchoring support for the prosthesis 100 in the user's body and is further configured with a concave surface 114 at its distal end to provide a ball and socket joint with the articulated column 106. As with the previous embodiments, the embodiment illustrated in Figures 7-9 may be implanted using conventional surgical techniques.

Preferably, the proximal end portion 102 is formed from a material such as silicon rubber which may be readily trimmed to provide a length and diameter configured to match the patient's corpora size. The distal end portion 104, preferably bullet- or cone-shaped, is adapted for the distal end of the penis.

The articulated column 106 which is positioned longitudinally between the proximal end portion 102 and the distal end portion 104 includes a plurality of alternating spheres 116 and cylindrical members 118. The cylindrical members 118 define concave surfaces 120 at their ends such that the adjoining concave surfaces 120 of the cylindrical members 118 and the spheres 116 form ball and socket joints 122. The concave surface 114 of the distal end portion 102 cooperates with one of the spheres 116 at the proximal end of the articulated column 106 to form a ball and socket joint therebetween. The spheres 116 and the cylindrical members 118 are journaled axially about the tension member 108. As previously discussed, it will be appreciated that in alternate embodiments of the present invention other elements might be utilized with slidable cooperating surfaces to form the articulated column 106 in keeping with the principles of the present invention.

In various embodiments, the spheres 116 might have indentations in the surfaces thereof and the cylindrical members 118 corresponding protrusions in the surfaces thereof. Accordingly, when the articulated column is compressed, the indentations and protrusions cooperate with each other to increase the overall friction of the ball and socket joints.

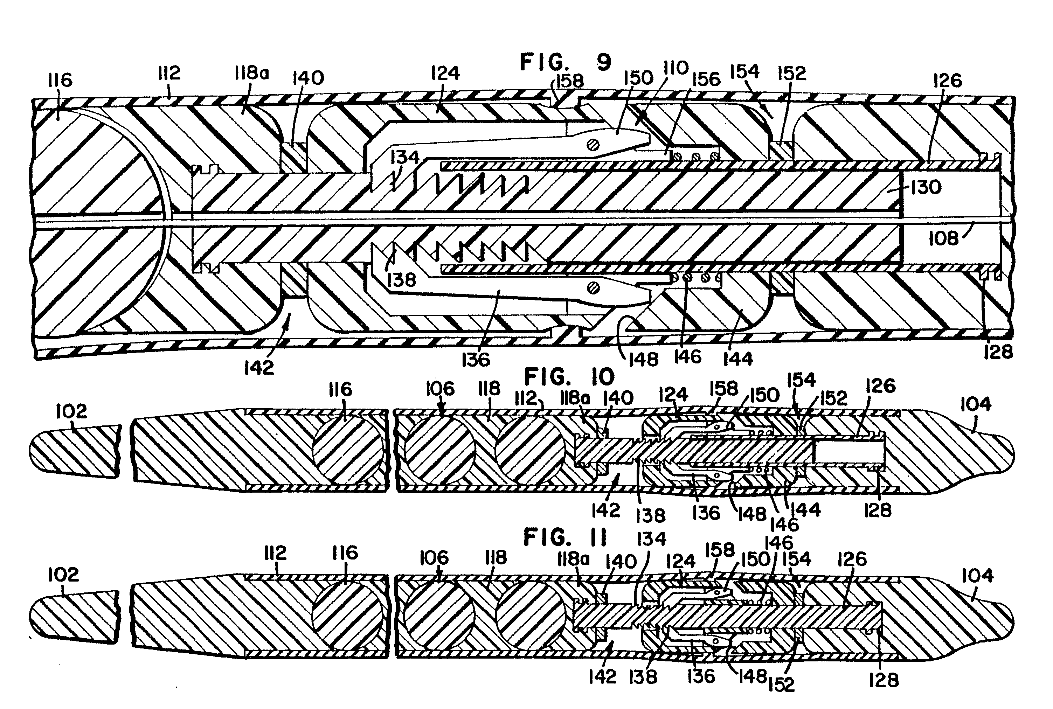

The switch apparatus 110 is positioned between the distal end portion 104 and the distal end of the articulated column 106. It will be appreciated that the switch apparatus 110 could also be located at other locations along the prosthesis. The switch apparatus 110 provides for control of axial elongation of the prosthesis 100 resulting in compression of the articulated column 106 and tensioning of the tension member 108. As illustrated in Figure 9, the switch apparatus 110 includes a retaining case 124 fixedly secured to a sleeve member 126 which extends axially into the distal end portion 104 where it is anchored by flange portions 128. The sleeve member 126 slidably receives a shaft member 130 which is secured at its proximal end to a modified cylindrical member 118a of the articulated column 106 by flange portions 132. As illustrated, the shaft 130 and the cylindrical member 118a are axially journaled to allow the tension member 108 to pass axially therethrough. The shaft 130 is formed with serrated engagement points 134 on its outer surface along at least a portion thereof. Retaining case 124 extends axially beyond the proximal end of the sleeve member 126 and is journaled to allow the shaft 130 to slide therethrough.

The retaining case 124 includes a pair of pivoting arms or pawls 136 with serrated teeth 138 which are configured to ratchet distally along serrations 134 of the shaft 130. A spacer member 140 fixedly secured on the shaft 130 between the articulated column 106 and the retaining case 124 has a lesser diameter than either the articulated column 106 or the retaining case 124 whereby an indentation or activation site 142 is formed which may be felt by touch. Transcutaneous radial pressure from a user's finger(s) at the activation site 142 is transduced to generally axial elongation of the switch apparatus 110 by causing a ratcheting action of the pawls 136 along the shaft 130 whereby the prosthesis 100 is positioned in an erect or rigid state.

A release member or collar portion 144 is axially, slidably positioned about the sleeve 126 between the retaining case 124 and the distal end portion 104. The release member 144 is separated from release ends 150 of the pawls 136 by a coil spring 146 positioned about the sleeve 126 and abutting a distal lip portion 156 of the retaining case 124. The release member 144 includes a beveled portion 148 which when forced axially in a proximal direction engages the release ends 150 of the pawls 136 thereby causing disengagement of the pawls 136 from the serrations 134 of the shaft 130. The spring 146 biases the collar portion 144 away from the release ends 150 of the pawls 136 to prevent the beveled portion 148 of the collar 144 from engaging the pawls 136 so as to accidentally cause disengagement of the pawls 136 from the shaft 130. However, upon manual application of sufficient force, the spring tension is overcome and the pawls 136 released. A spacer 150, fixedly secured to the sleeve 126 between the collar portion 144 and the distal end portion 104 and further being of less diameter than the collar portion 144 and the distal end portion 104, provides a readily discernable release site 154 which may be palpated to overcome the bias of the spring 146 thereby releasing the prosthesis 100 from its erect, rigid state and placing the prosthesis 100 in a flaccid state.

As with previous engagements, the external cylindrical sheath 112 is preferably formed from a physiologically inert material such as silicon rubber, Dacron, expanded PTFE, etc. and encases the prosthesis 100 in a fluid - tight manner. In the embodiment illustrated in Figures 7-9, the sheath is attached to the retaining case 124 at a sheath attachment portion 158.

In use, the implanted prosthesis is normally maintained in an untensioned state which does not interfere with bending or moving the penis. The pawl teeth 138 engage the shaft teeth 134 at the proximal end of the shaft 130, as generally illustrated in Figure 7. In this condition, the prosthesis 100 is at its minimum length and the tension member 108 is untensioned. This lack of tension allows the surfaces of the ball and socket joints 122 of the articulated column 106 to slide relative to each other under a low friction condition so as to move freely, maintaining flexibility to the prosthesis in the penis as shown in Figure 7.

To provide an erection, the user manually applies repetitive palpations at the activation site 142. The radial force is transduced to axially elongation of the prosthesis by causing a ratcheting effect and relative translational movement between the pawls 136 and the shaft 130.

When the device is actuated, the shaft 130 is displaced proximally, sliding away from a distal end of the sleeve 126 as illustrated in Figure 8. This increases the length of the prosthesis 100 and the tension member 108 is accordingly placed under tension. Furthermore, the articulated column 106 is compressed due to displacement of the shaft 130. Accordingly, the increased tension in the tension member 108 and compression of the articulated column 106 decreases the separation between each of the ball and socket joints 122, thereby increasing frictional resistance to movement. Friction between the sheath 112 and the internal articulated column 106 along the longitudinal circumference also may contribute to the overall rigidity of the prosthesis 100.

It has been found that an axial elongation of two to six milimeters will generate adequate tension to maintain an erection.

Restoration of penile flexibility is achieved by finger palpations at the deactivation site 154. The application of radial pressure causes the release member 144 to move proximally against the spring 146 until the beveled portion 148 contacts the pawl release ends 150 which disengage the pawls 136 from the shaft 130. Flaccidity is restored as the shaft 130 slides distally along the sleeve 126 such that tension in the tension member 108 is reduced.

It will be appreciated that the prosthesis may be deactivated and activated repetitively numerous times. Furthermore, once positioned in either a flaccid state or a rigid state, the prosthesis 100 will remain in the state where currently positioned until manually changed thereby providing for user volitional control. In addition, the embodiments shown in Figures 7-9 provide for varying degrees of rigidity as the pawls 136 ratchet distally along the shaft 130.

Yet another embodiment of the present invention is shown in Figure 10 which does not utilize an axial tension member. In this embodiment, the sheath 112 is utilized as the tensioning member.

Still yet another embodiment of the present invention is shown in Figure 11. Once again, the sheath 112 is utilized as the tensioning member as opposed to an axial tension member; however, the embodiment shown in Figure 11 is generally of fixed length as the distal end portion 104 is attached directly to the shaft 130. Accordingly, as the switch apparatus 110 moves distally on the shaft 130, the sheath 112 is placed in tension but the overall length of the prosthesis changes very little if at all. In this embodiment, the sheath 112 is preferably relatively unstretchable, as the sheath 112 may include woven layers to provide adequate strength to resist stretching as the prosthesis is operated.

It is to be understood, however, even though numerous advantages and characteristics of the invention have been set forth in the foregoing description, together with details of the structure and function of the invention, the disclosure is illustrative only, and changes may be made in detail, especially in matters of shape, size and arrangement of parts within the principles of the present invention, to the full extent indicated by the broad general meaning of the terms in which the appended claims are expressed.

## Claims

1. A pencile prosthesis, comprising an articulated column (26, 106) of segments (50) engaging each other to form slidable joints, an outer elongated sheath (28, 112) of a physiologically inert and pliable material extending longitudinally of the prosthesis and connected to the prosthesis proximate to both ends of the prosthesis for holding the segments of the articulated column to interengagement at adjacent ends thereof, the prosthesis being adjustable between flaccid and rigid conditions, **characterized** in that axially elongatable and shortenable switch means (30, 110) within said sheath (28, 112) axially disposed intermediate of said articulated column (26, 106) and of one end of the prosthesis to be axially displaced between first and second operative states for adjusting the prosthesis between said flaccid and rigid conditions, is arrestable by locking means (66, 68; 134, 136) in said first and second operative states and operatively connected, at one end of the column (26, 106) of segments (50), to the sheath (28, 112) or to a tension member (32, 108) extending through the segments to put the sheath (28, 112) or the tension member (32, 108), respectively, in said first state into a relaxed condition allowing the segments to articulate for the flaccidity of the prosthesis, in said second state into a tensioned condition providing increased frictional resistance between the segments by compression of the articulated column for the rigidity of the prostehsis.

2. The penile prosthesis of claim 1, wherein said locking means (134, 136) includes ratchet means, said ratchet means comprising a central toothed shaft (130) aligned with and anchored to said articulated column (106) and further comprising pawl means (136) fixedly secured proximate the one end (104) of the prosthesis for ratcheting axially along said toothed shaft, and pawl release means (144) to release said pawl means from engagement with said toothed shaft, wherein ratcheting action between said pawl means and said toothed shaft causes axial elongations of said switch means whereby causing compression of said articulated column (106) and tensioning of said tension means (108), thereby increasing the rigidity of said articulated column, said pawl release means causing axial shortening of said switch means thereby decreasing the rigidity of said articulated column.

3. The penile prosthesis of claim 2, wherein said segments comprise a plurality of alternating spherical members (116) and cylindrical members (118), each of said cylindrical members having concave surfaces (122) at each end constructed and arranged for forming a slidable joint with said spherical members.

4. The penile prosthesis of claim 1, wherein said switch means (110) includes an activation site (142), application of radial pressure at said activation site by a user being transduced by said switch means to axial elongation of said switch means, said activation site being readily discernable by touch.

5. The penile prosthesis of claim 1, wherein said segments (50) have radiused mating surfaces enclosed within said outer elongated sheath (28) and being held in place with reference to adjacent segment by said sheath.

6. The penile prosthesis of claim 1, wherein said switch means (30) comprising biasing means (64) for providing a biasing force axially in a first direction away from said articulated column (26), an axially slidable portion including a rotary member (66) and a non-rotary member (68) being biased in the first direction away from said articulated column by said biasing means, said biasing means being disposed intermediate of said rotary member and an end portion of said articulated column, said tension member(32) being interconnected proximate the one end to said axially slidable portion, said axially slidable portion being slidable between a first position and a second position further removed from the one end (104) of the prosthesis than said first position, and means (74, 76, 78) for alternately locking said rotary member at said second position, said tension member being relatively untensioned when said rotary member is positioned at said second position whereby the prosthesis is relatively flexible, said tension member being placed under tension and said articulated column being compressed when said rotary member is positioned at said first position whereby the prosthesis is relatively rigid, and wherein said tension member cooperates with said switch means to displace said axially slidable portion in a second direction toward said articulated column upon bending of the prosthesis which places the tension member in sufficient tension to overcome the force of the biasing means.

**Revendications**

1. Prothèse pour pénis comprenant une colonne (26, 106) articulée de tronçons (50) s'engageant les uns avec les autres pour constituer des jointures glissantes, une gaine extérieure allongée (28, 112) en matière physiologiquement inerte et souple s'étendant dans le sens longitudinal de la prothèse et raccordée à celle-ci à proximité des deux extrémités de cette prothèse pour tenir les tronçons de la colonne articulée en inter-engagement à leurs extrémités voisines, la prothèse étant réglable entre un état flasque et un état rigide, caractérisée en ce qu'un moyen (30, 110) de changement d'état allongeable et raccourcissable en sens axial à l'intérieur de la gaine (28, 112) disposé en sens axial entre la colonne articulée (26, 106) et une extrémité de la prothèse pour être déplacé en sens axial entre un premier et un second état fonctionnel pour le réglage de la prothèse entre l'état flasque et l'état rigide, est verrouillable par un moyen de verrouillage (66, 68 ; 134, 136) à ses premier et second états et raccordé fonctionnellement, à une extrémité de la colonne (20, 106) des tronçons (50), à la gaine (28, 112) ou à un organe de mise en tension (32, 108) s'étendant à travers les tronçons pour mettre la gaine (28, 112) ou l'organe (32, 108) de mise en tension, respectivement, dans le premier état en situation de relâchement permettant aux tronçons de s'articuler pour l'état flasque de la prothèse, dans le second état en situation de tension créant une résistance accrue au frottement entre les tronçons par compression de la colonne articulée en vue de la rigidité de la prothèse.

2. Prothèse pour pénis de la revendication 1, dans laquelle le moyen de verrouillage (134 ; 136) comprend un organe à cliquet, cet organe à cliquet comprenant un axe central denté (130) aligné avec la colonne articulée (106) et ancré à celle-ci et comprenant en plus un cliquet (136) fixé fermement à proximité d'une extrémité (104) de la prothèse pour exercer un effet de cliquet en sens axial le long de l'axe denté, et un moyen (144) de relâchement du cliquet pour libérer le cliquet de son engagement avec l'axe denté, de sorte que l'action de cliquet entre le cliquet et l'axe denté provoque des élongations en sens axial du moyen de changement d'état provoquant de cette façon la compression de la colonne articulée (106) et la mise en tension de l'organe (108) de tension, accroissant ainsi la rigidité de la colonne articulée, le moyen de relâchement du cliquet provoquant un raccourcissement en sens axial de ce moyen de changement d'état et diminuant de cette façon la rigidité de la colonne articulée.

3. Prothèse pour pénis de la revendication 2, dans laquelle les tronçons comprennent une pluralité d'éléments sphériques (116) et d'organes cylindriques (118) alternant les uns avec les autres, chacun des organes cylindriques ayant des surfaces concaves (122) à chaque extrémité, réalisées et arrangées pour composer une jointure glissante avec les organes sphériques.

EP 0 139 745 B1

4. Prothèse pour pénis de la revendication 1, dans laquelle le moyen (110) de changement d'état comprend un endroit d'activation (142), l'application par un utilisateur d'une pression radiale à cet endroit d'activation étant traduite par le moyen de changement d'état en une élongation en sens axial de ce moyen, cet endroit d'activation étant facilement discernable au toucher.

5. Prothèse pour pénis de la revendication 1, dans laquelle les tronçons (50) ont des surfaces arrondies s'ajustant ensemble encloses à l'intérieur de la gaine allongée extérieure (28) et tenues en place par ladite gaine par rapport aux tronçons voisins.

6. Prothèse pour pénis de la revendication 1, dans laquelle le moyen (30) de changement d'état comprend un moyen de rappel (64) pour fournir une force de rappel en sens axial dans une première direction d'éloignement par rapport à la colonne articulée (26), une partie coulissante en sens axial comprenant un organe tournant (66) et un organe non tournant (68) étant rappelée dans la première direction d'éloignement par rapport à la colonne articulée par ledit moyen de rappel, ce dernier étant disposé entre l'organe tournant et une partie extrême de la colonne articulée, l'organe (32) de mise en tension étant interconnecté à proximité de ladite partie extrême à la partie coulissante en sens axial, cette partie coulissante en sens axial pouvant glisser entre une première position et une seconde position éloignée davantage de ladite partie extrême (104) de la prothèse que la première position, et un moyen (74, 76, 78) pour verrouiller en alternance l'organe tournant à la seconde position, l'organe de mise en tension étant relativement sans tension quand l'organe tournant est placé à la seconde position de sorte que la prothèse est relativement flexible, cet organe de mise en tension étant placé sous tension et la colonne articulée étant comprimée quand l'organe tournant est placé à la première position de sorte que la prothèse est relativement rigide, et dans laquelle le moyen de mise en tension coopère avec le moyen de verrouillage pour déplacer ladite partie glissant en sens axial à une seconde position en direction de la colonne articulée à la suite d'une flexion de la prothèse qui met le moyen de mise en tension en tension suffisante pour que soit surmontée la force du moyen de rappel.

**Patentansprüche**

1. Penisprothese, umfassend eine gelenkig gegliederte Säule (26, 106) aus Segmenten (50), die unter Bildung von verschiebbaren Gelenkverbindungen miteinander in Eingriff stehen, und eine äußere, langgestreckte Hülle (28, 112) aus einem physiologisch inerten und biegsamen Werkstoff, die sich in Längsrichtung der Prothese erstreckt und mit dieser im Bereich ihrer beiden Enden verbunden ist, um die Segmente der gelenkig gegliederten Säule an ihren benachbarten Enden in gegenseitigem Eingriff zu halten, wobei die Prothese zwischen schlaffen und starren Zuständen verstellbar ist, dadurch gekennzeichnet, daß ein axial verlängerbares und verkürzbares Schaltmittel (30, 110), das innerhalb der Hülle (28, 112) axial zwischen der gelenkig gegliederten Säule (26, 106) und dem einen Ende der Prothese zwischen ersten und zweiten Betriebslagen für Verstellung der Prothese zwischen den schlaffen und starren Zuständen axial verschiebbar angeordnet ist, durch eine Arretiereinrichtung (66, 68; 134, 136) in erster und zweiter Betriebslage arretierbar und am einen Ende der Säule (26, 106) aus Segmenten (50) betrieblich oder wirkungsmäßig mit der Hülle (28, 112) oder einem sich durch die Segmente erstreckenden Spannelement (32, 108) verbunden ist, um die Hülle (28, 112) bzw. das Spannelement (32, 108) in der ersten (Betriebs-)Lage in einen entspannten Zustand, in welchem sich die Segmente für Schlaffheit der Prothese gelenkig verschwenken können, und in der zweiten (Betriebs-) Lage in einen gespannten oder gestrafften Zustand, in welchem erhöhter Reibungswiderstand zwischen den Segmenten durch Zusammendrückung der gelenkig gegliederten Säule hervorgebracht wird, für Starrheit oder Steifheit der Prothese zu bringen.

2. Penisprothese nach Anspruch 1, dadurch gekennzeichnet, daß die Arretiereinrichtung (134, 136) Gesperremittel mit einem zentralen, verzahnten Schaft (130), der mit der gelenkig gegliederten Säule (106) ausgefluchtet und an dieser verankert ist, und mit im Bereich des einen Endes (104) der Prothese sicher befestigten Sperrklinkenmitteln (136), die axial längs des verzahnten Schafts (mit einer Ratschenwirkung) einrastend verschiebbar sind, sowie Sperrklinkenausrastmittel (144) zum Ausrasten der Sperrklinkenmittel aus dem Eingriff mit dem verzahnten Schaft aufweist, wobei die Ratscheneinrastwirkung zwischen den Sperrklinkenmitteln und dem verzahnten Schaft axiale Verlängerungen des Schaltmittels unter Zusammendrückung der gelenkig gegliederten Säule (106) und Spannung des Spannelements

10

(108) herbeiführt, um damit die Starrheit der gelenkig gegliederten Säule zu erhöhen, und wobei das Sperrklinkenausrastmittel eine axiale Verkürzung des Schaltmittels und damit eine Verringerung der Starrheit der gelenkig gegliederten Säule herbeiführt.

3. Penisprothese nach Anspruch 2, dadurch gekennzeichnet, daß die Segmente eine Anzahl von einander abwechselnden sphärischen Elementen (116) und zylindrischen Elementen (118) umfassen, wobei jedes der zylindrischen Elemente an jedem Ende konkave Flächen (122) einer solchen Ausbildung und Anordnung, daß sie eine verschiebbare Verbindung mit den sphärischen Elementen bilden, aufweist.

4. Penisprothese nach Anspruch 1, dadurch gekennzeichnet, daß das Schaltmittel (110) eine Aktivierstelle (142) aufweist, wobei ein durch einen Benutzer radial auf die Aktivierstelle ausgeübter Druck durch das Schaltmittel in eine axiale Verlängerung des Schaltmittels umgesetzt wird, und daß die Aktivierstelle bei Berührung leicht wahrnehmbar ist.

5. Penisprothese nach Anspruch 1, dadurch gekennzeichnet, daß die Segmente (50) verrundete, innerhalb der äußeren, langgestreckten Hülle (28) eingeschlossene Paßflächen aufweisen und durch die Hülle in ihrer Lage in bezug auf ein benachbartes Segment gehalten sind.

6. Penisprothese nach Anspruch 1, dadurch gekennzeichnet, daß das Schaltmittel (30) ein Vorbelastungsmittel (64) zur Lieferung einer axial in einer ersten Richtung von der gelenkig gegliederten Säule (26) hinweg wirkenden Vorbelastungskraft, einen axial verschiebbaren Abschnitt mit einem drehbaren Element (66) und einem drehfesten Element (68), die durch das Vorbelastungsmittel in der ersten Richtung von der gelenkig gegliederten Säule hinweg vorbelastet sind, wobei das Vorbelastungsmittel zwischen dem drehbaren Element und einem Endabschnitt der gelenkig gegliederten Säule angeordnet ist, das Spannelement (32) im Bereich des einen Endes mit dem axial verschiebbaren Abschnitt verbunden ist· und der axial verschiebbare Abschnitt zwischen einer ersten Stellung und einer zweiten, weiter als die erste Stellung von dem einen Ende (104) der Prothese entfernten Stellung verschiebbar ist, sowie Mittel (74, 76, 78) zum abwechselnden Verriegeln des drehbaren Elements in der zweiten Stellung aufweist, wobei das Spannelement vergleichsweise ungespannt ist, wenn das drehbare Element in der zweiten Stellung positioniert ist, so daß die Prothese vergleichsweise biegsam ist, und das Spannelement unter Zugspannung gelangt und die gelenkig gegliederte Säule zusammengedrückt ist, wenn das drehbare Element in der ersten Stellung positioniert ist, so daß die Prothese vergleichsweise starr oder steif ist, und daß das Spannelement mit dem Schaltmittel zusammenwirkt, um den axial verschiebbaren Abschnitt in einer zweiten Richtung zur gelenkig gegliederten Säule hin zu verschieben, wenn die Prothese gebogen wird, wodurch das Spannelement unter eine ausreichende Zugspannung zur Überwindung der Kraft des Vorbelastungsmittels gebracht wird.

FIG. 1

FIG. 2

FIG. 6

FIG. 3

FIG. 4

FIG. 5

FIG. 7

FIG. 8

14

FIG. 9

FIG. 10

FIG. 11